(19) Europäisches Patentamt
European Patent Office
Office européen des brevets

(11) Publication number: **0 272 824**
**A2**

(12) **EUROPEAN PATENT APPLICATION**

(21) Application number: **87310642.1**

(22) Date of filing: **03.12.87**

(51) Int. Cl.[4]: **C07D 213/64** , C07D 213/73 , A01N 43/40

(30) Priority: **24.12.86 GB 8630847**

(43) Date of publication of application: **29.06.88 Bulletin 88/26**

(84) Designated Contracting States: **AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Perrior, Trevor Robert**
**35 Kesteven Way**
**Wokingham Berkshire(GB)**
Inventor: **Tapolczay, David Joszef**
**9 Foresters Square**
**Bracknell Berkshire(GB)**

(74) Representative: **Hardman, Carol Pauline et al**
**Imperial Chemical Industries PLC Legal Department: Patents PO Box 6 Bessemer Road**
**Welwyn Garden City Herts, AL7 1HD(GB)**

(54) **Chemical compounds.**

(57) Pesticidal compounds of formula (I)

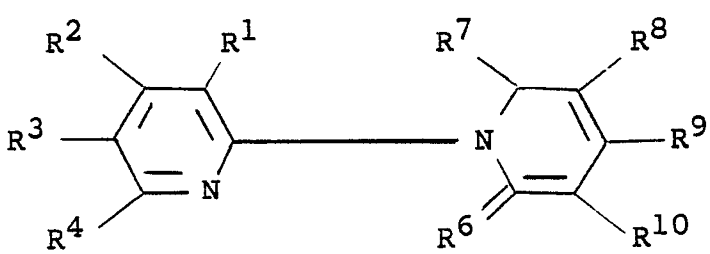

(I)

wherein $R^1$, $R^2$, $R^3$ and $R^4$ are independently selected from hydrogen, cyano, halogen, lower alkyl optionally substituted by halogen, lower alkoxy optionally substituted by halogen, lower alkenyl optionally substituted by halogen; amino and mono-or di-(lower alkyl) amino;
$R^6$ is oxygen or sulphur;
$R^7$, $R^8$ and $R^{10}$ are independently selected from hydrogen, halogen, cyano, lower alkyl optionally substituted by halogen, lower alkoxy optionally substituted by halogen, lower thioalkoxy optionally substituted by halo; and
$R^9$ is lower alkyl substituted by halogen or lower alkenyl optionally substituted by halogen.

The preparation of these compounds together with their use in combatting insect, acarine and nemotode pests is also described.

EP 0 272 824 A2

## CHEMICAL COMPOUNDS

This invention relates to novel pyridone derivatives useful as insecticidal agents.

Japanese Kokai No 60038363 discloses an insecticidal pyridyl pyridone derivative of formula (A).

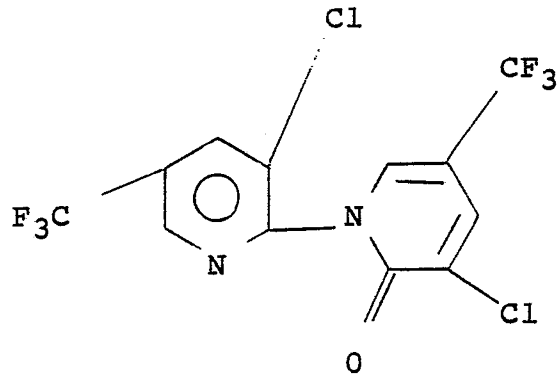

(A)

Japanese Kokai No 61072754 discloses further insecticidal pyridyl pyridone derivatives of general formula (B)

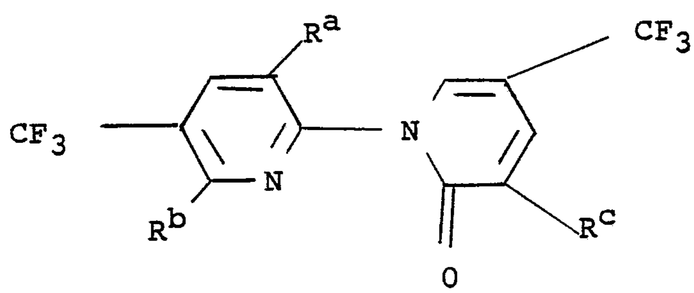

(B)

wherein $R^a$ is fluorine or chlorine;

$R^b$ is hydrogen fluorine or chlorine; and

$R^c$ is hydrogen, fluorine, chlorine, bromine or cyano.

Japanese Kokai No 61186.363 discloses the preparation of compounds of formula (C)

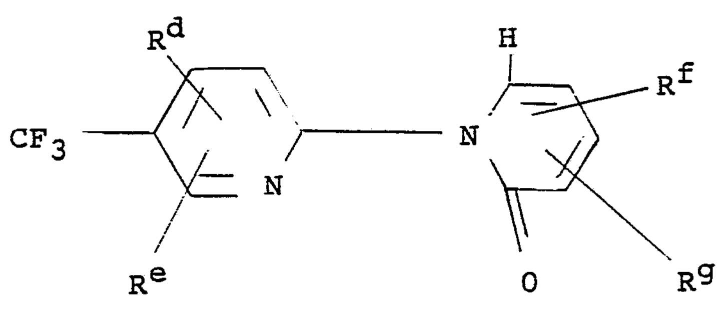

(C)

wherein $R^d$ and $R^e$ are independently hydrogen, halogen, lower alkyl, trifluoromethyl or cyano, and $R^f$ and R $^g$ are independently hydrogen, halogen, lower alkyl, nitro, trifluoromethyl or cyano.

The applicants have found further pyridyl pyridine derivatives which show improved insecticidal properties over the compounds of the prior art.

According to the present invention, there is provided a compound of formula (I)

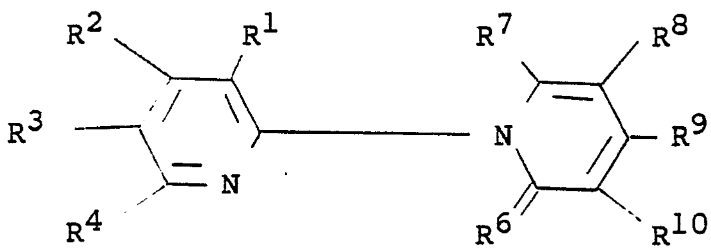

(I)

wherein $R^1$, $R^2$, $R^3$ and $R^4$ are independently selected from hydrogen, halogen, cyano, lower alkyl optionally substituted by halogen, lower alkoxy optionally substituted by halogen, lower alkenyl optionally substituted by halogen, nitro, amino and mono-or di-(lower alkyl)amino;

$R^6$ is oxygen or sulphur;

$R^7$, $R^8$ and $R^{10}$ are independently selected from hydrogen, halogen, cyano, lower alkyl optionally substituted by halogen, lower alkoxy optionally substituted by halogen, and lower thioalkoxy optionally substituted by halogen; and

$R^9$ is lower alkyl substituted by halogen or lower alkenyl optionally substituted by halogen.

As used herein the term "lower" used in relation to alkyl or alkoxy groups means groups having from 1 to 6 carbon atoms preferably from 1 to 3 carbon atoms and when used in relation to alkenyl groups means groups having from 2 to 6 carbon atoms, preferably 2 or 3 carbon atoms.

As used herein the term "halogen" includes fluorine, chlorine, bromine and iodine.

Suitably $R^1$, $R^2$, $R^3$ and $R^4$ are selected from hydrogen, chlorine, bromine, nitro, amino or trifluoromethyl.

Suitable examples of $R^1$ include chlorine, bromine, nitro, or amino.

A suitable example of $R^2$ is hydrogen.

A suitable example of $R^3$ is trifluoromethyl.

Suitable examples of $R^4$ include hydrogen and chlorine, preferably hydrogen.

Preferably $R^6$ is oxygen.

Suitably $R^7$, $R^8$ and $R^{10}$ are selected from hydrogen, chloro, bromo or trifluoromethyl.

Preferably $R^7$ and $R^{10}$ are hydrogen.

Examples of suitable groups $R^8$ include hydrogen, chlorine, bromine, and trifluoromethyl.

Preferably $R^9$ is lower alkyl substituted by fluorine or chlorine such as trifluoromethyl or chlorodifluoromethyl.

Preferably $R^9$ is trifluoromethyl.

Thus a preferred sub-group of compounds of the invention are those of formula IA

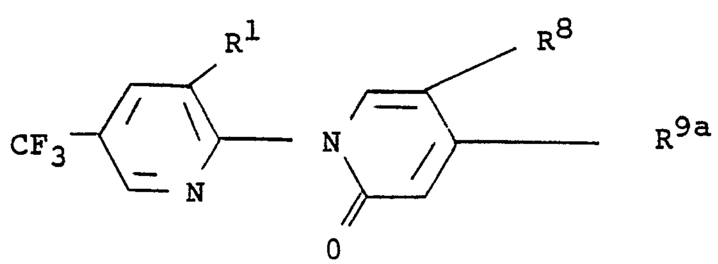

(IA)

wherein $R^1$ and $R^8$ are as hereinbefore defined and $R^{9a}$ is lower alkyl substituted by halogen.

Specific examples of compounds of formula I are set out in Table I.

TABLE I

| COMPOUND | $R^1$ | $R^2$ | $R^3$ | $R^4$ | $R^6$ | $R^7$ | $R^8$ | $R^9$ | $R^{10}$ |
|---|---|---|---|---|---|---|---|---|---|
| 1 | Cl | H | $CF_3$ | H | 0 | H | H | $CF_3$ | H |
| 2 | Cl | H | $CF_3$ | H | 0 | H | Cl | $CF_3$ | H |
| 3 | Cl | H | $CF_3$ | H | 0 | H | Br | $CF_3$ | H |
| 4 | Cl | H | $CF_3$ | H | 0 | H | $CF_3$ | $CF_3$ | H |
| 5 | Cl | H | $CF_3$ | Cl | 0 | H | Br | $CF_3$ | H |
| 6 | Cl | H | $CF_3$ | H | 0 | H | H | $CF_2Cl$ | H |
| 7 | Br | H | $CF_3$ | H | 0 | H | H | $CF_3$ | H |
| 8 | $NO_2$ | H | $CF_3$ | H | 0 | H | H | $CF_3$ | H |
| 9 | $NH_2$ | H | $CF_3$ | H | 0 | H | H | $CF_3$ | H |

The compounds of formula I may be prepared by reacting a compound of formula (II):

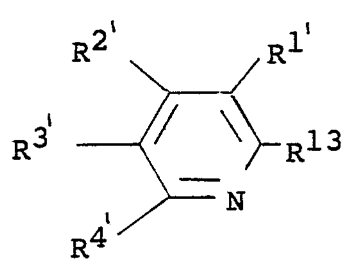

(II)

wherein $R^{1'}$, $R^{2'}$, $R^{3'}$ and $R^{4'}$ are $R^1$, $R^2$, $R^3$ and $R^4$ as hereinbefore defined or a precursor thereof and $R^{13}$ is a leaving group such as halo, with a compound of formula (III)

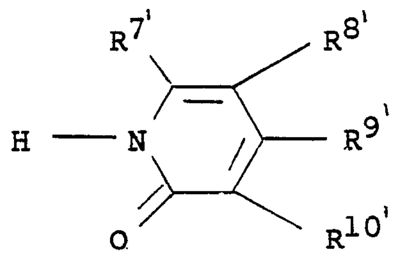

(III)

where $R^{7'}$, $R^{8'}$, $R^{9'}$ and $R^{10'}$ are $R^7$, $R^8$, $R^9$ or $R^{10}$ as hereinbefore defined or a precursor thereof; and thereafter if desired carrying out one or more of the following steps;

(i) converting a group $R^{1'}$, $R^{2'}$, $R^{3'}$, $R^{4'}$, $R^{7'}$, $R^{8'}$, $R^{9'}$, $R^{10'}$ to $R^1$, $R^2$, $R^3$, $R^4$, $R^7$, $R^8$, $R^9$ or $R^{10}$ respectively; or

(ii) converting the carbonyl group to =S.

The reaction is suitably carried out in the presence of a solvent and a base. The base may be for example an alkali metal hydride, an alkali metal alkoxide or an alkali metal carbonate, and the solvent may be a hydrocarbon solvent, such as petroleum ether, an alcohol or an aprotic polar solvent such as dimethylformamide or dimethylacetamide. Suitable halo groups $R^{13}$ include fluoro, chloro, bromo or iodo. If necessary an appropriate catalyst such as a crown ether or copper can be added depending upon the precise nature of $R^{13}$. Further details of the process for preparation of the compounds may be ascertained from the Examples set out hereinafter.

Optional step (ii) above may be carried out by reacting compounds of formula (I) wherein $R^6$ is oxygen with a thiolating agent such as phosphorus pentasulphide. The reaction is suitably carried out in an organic solvent such as pyridine at elevated temperatures of from 50°C to 150°C.

As used herein the term "precursor" applies to a group which can be converted to a chemical group $R^1$, $R^2$, $R^3$, $R^4$, $R^7$, $R^8$, $R^9$ or $R^{10}$ by standard chemical techniques. A particularly useful precursor of this type is the formyl group. This group can be converted to various optionally substituted alkyl, optionally substituted alkenyl, optionally substituted alkynyl, and cyano groups by known chemical techniques.

For example compounds of formula (I) where $R^9$ is optionally substituted alkenyl can be prepared by reacting a compound of formula (IV)

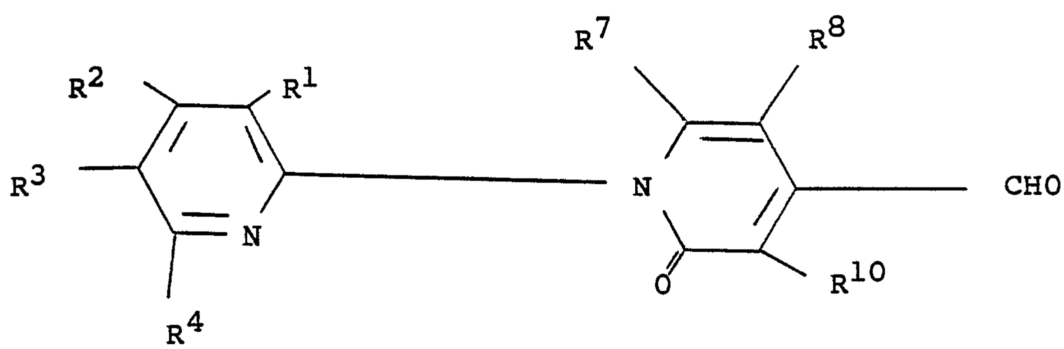

(IV)

wherein $R^1$, $R^2$, $R^34$, $R^4$, $R^7$, $R^8$ and $R^{10}$ are as hereinbefore defined with an appropriate substituted alkane in the presence of zinc and triphenyl phosphine under conventional Wittig reaction conditions.

A further useful precursor group, is ethoxycarbonyl. This group can be readily converted to various hydroxy alkyl groups such as hydroxy methyl or 1-methyl-1-hydroxy ethyl by standard techniques such as by reduction or using Grignard reactions. The hydroxy alkyl groups can be converted directly to compounds of the invention, for example by halogenation or they may be converted to a second precursor such as formyl which is discussed above.

Compounds of formula (I) can be converted to other compounds of formula (I) having different substituent R groups by conventional methods if desired. For example, when one of $R^1$, $R^2$, $R^3$ or $R^4$ is fluoro, it can be converted to an alkoxy group by reaction with an appropriate alkoxy anion of formula $R^{14}0$- where $R^{14}$ is lower alkyl. Anions of formula $R^{14}0$-may be prepared by dissolving sodium metal in an alcohol

of formula $R^{14}OH$ suitably at moderate temperatures of from 0 to 100°C, preferably at room temperature.

An additional where $R^1$, $R^2$, $R^3$ or $R^4$ is nitro group, it can be converted to amino as illustrated hereinafter.

Compounds of formula (IV) can be prepared by oxidation of a compound of formula (V)

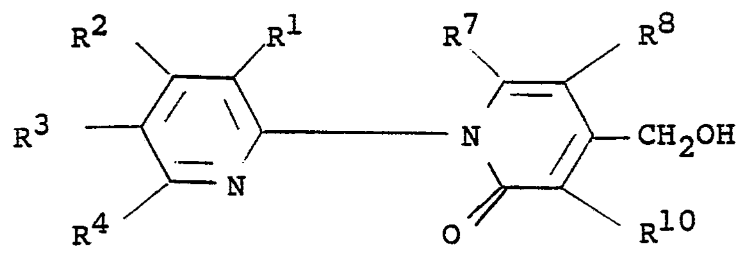

(V)

wherein $R^1$, $R^2$, $R^3$, $R^4$, $R^7$, $R^8$ and $R^{10}$ are as hereinbefore defined. The oxidation is suitably carried out using an oxidising agent for example using Swern oxidation procedures. The reaction is suitably carried out in an organic solvent such as dichloromethane in the presence of a base. Low temperatures for example from -100°C to 0°C are suitably employed.

Compounds of formula (V) can be prepared by reduction of a compound of formula (VI)

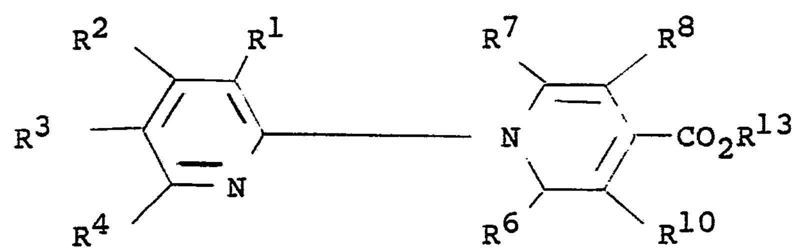

(VI)

wherein $R^1$, $R^2$, $R^3$, $R^4$, $R^6$, $R^7$, $R^8$ and $R^{10}$ are as hereinbefore defined and $R^{13}$ is lower alkyl optionally substituted by halogen; using a reducing agent such as lithium borohydride. The reaction is suitably carried out in an inert organic solvent at temperatures of from 0-150°C. A suitable solvent is tetrahydrofuran.

Compounds of formula (II) are largely known compounds or they can be prepared from known compounds by conventional methods. However certain of these compounds are novel and these form part of the invention.

Therefore, in a further aspect of the invention there is provided a compounds of formula (VII)

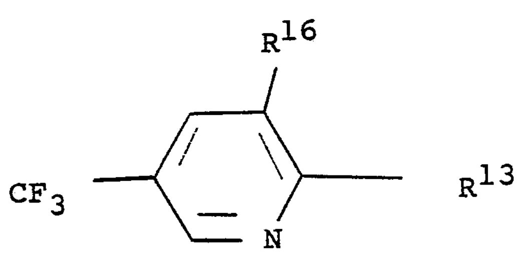

(VII)

wherein $R^{13}$ is a hereinbefore defined and $R^{16}$ is bromo or nitro.

These compounds can be prepared by halogenation of a compound of formula (VIII)

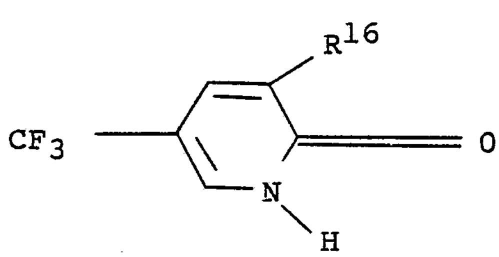

(VIII)

wherein $R^{16}$ is as hereinbefore defined.

Halogenation is suitably effected using a halogenating agent such as phosphorus oxcychloride and phosphorus pentachloride.

The compounds of formula (VIII) where $R^{16}$ is nitro is also novel.

This compound can be prepared by nitration of a compound of formula (IX)

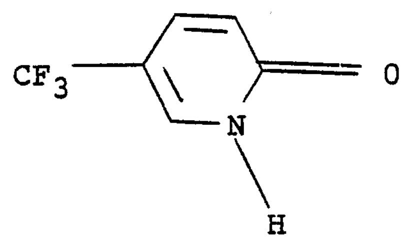

(IX)

using a nitrating agent such as a mixture of concentrated nitric and concentrated sulphuric acids.

Examples of these reactions are given hereinafter by way of illustration.

Compounds of formula (III) are largely known compounds or can be prepared from known compounds (see for example EP-A-216541).

Further such compounds are compounds of formula (X)

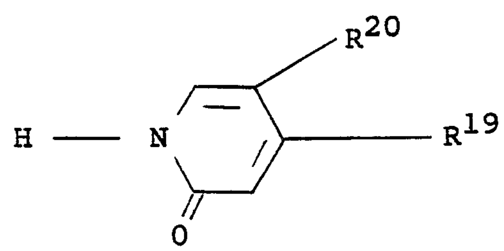

(X)

wherein $R^{19}$ is trihalomethyl and $R^{20}$ is an electrophilic group $R^{8'}$ as hereinbefore defined. These compounds are described in our copending European Application No 87307251.6.

Particular examples of $R^{19}$ include trifluoromethyl.

Particular examples of $R^{20}$ include nitro, lower thioalkoxy optionally substituted by halogen such as trifluoromethylthio, formyl, and lower alkyl as methyl or butyl optionally substituted by halogen.

Compounds of formula (X) wherein $R^{20}$ is an electrophilic group other than nitro can be prepared by reacting a compound of formula (XI)

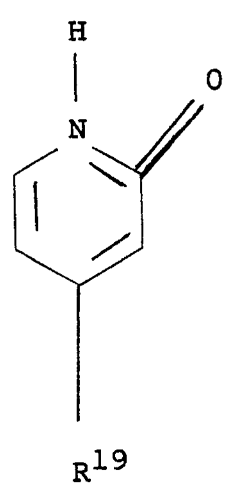

(XI)

wherein $R^{19}$ is as hereinbefore defind with a base and a compound

$$R^{20} \text{———} R^{21}$$

wherein $R^{21}$ is a leaving group such as halogen.

The base used in the reaction is preferably a combination of sodium hydride followed by t-butyllithium. Suitably 2 equivalents of base are employed. The reaction is preferably carried out in an inert organic solvent such as tetrahydrofuran.

Compounds of formula (X) wherein $R^{20}$ is nitro can be prepared by nitration of a compound of formula (XII) under standard conditions.

The compounds of formula I may be used to combat and control infestations of insect pests and also other invertebrate pests, for example, acarine pests and nematodes. The insect and acarine pests which may be combatted and controlled by the use of the invention compounds include those pests associated with agriculture (which term includes the growing of crops for food and fibre products), horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise an insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters of sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl-and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents.

Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydrofurfuryl alcohol (THFA).

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogenous for a sufficient time to enable them to be applied by conventional spray equipment. The concentrates may contain 10-85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient (approximately equivalent to from 5-2000g/ha) is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compounds of the invention may be the sole active ingredient of the composition or they may be

admixed with one or more additional active ingredients such as insecticides, insecticide synergists, herbicides, fungicides or plant growth regulators where appropriate. Suitable additional active ingredients for inclusion in admixture with the compounds of the invention may be compounds which will broaden the spectrum of activity of the compounds of the invention or increase their persistence in the location of the pest. They may synergise the activity of the compound of the invention or complement the activity for example by increasing the speed of effect, improving knockdown or overcoming repellency. Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following:

a) Pyrethroids such as permethrin, esfenvalerate, deltamethrin, cyhalothrin in particular, cyhalothrin, biphenthrin, fenpropathrin, cyfluthrin, tefluthrin, fish safe pyrethroids for example ethofenprox, natural pyrethrin, tetramethrin, s-bioallethrin, fenfluthrin, prallethrin and 5-benzyl-3-furylmethyl-(E)-(-R,3S)-2,2-dimethyl-3-(2-oxothiolan-3-ylidenemethyl) cyclopropane carboxylate;

b) Organophosphates such as profenofos, sulprofos, methyl parathion, azinphos-methyl, demeton-s-methyl, heptenophos, thiometon, fenamiphos, monocrotophos, profenophos, triazophos, methamidophos, dimethoate, phosphamidon, malathion, chloropyrifos, phosalone, fensulfothion, fonofos, phorate, phoxim, pyrimiphosmethyl, fenitrothion or diazionon;

c) Carbamates (including aryl carbamates) such as pirimicarb, cloethocarb, carbofuran, ethiofencarb, aldicarb, thiofurox, carbosulfan, beniocarb, fenobucarb, propoxur or oxamyl;

d) Benzoyl ureas such as triflumeron, or chlorofluazuron;

e) Organic tin compounds such as cyhexatin, fenbutatin oxide, azocyclotin;

f) Macrolides such as avermectins or milbemyins, for example such as avamectin, avermectin, and milbemycin;

g) Hormones such as pheromones;

h) Organochlorine compounds such as benzene hexachloride, DDT, chlordane or dieldrin.

i) Amidines, such as chlordimeform or amitraz.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin can be employed. Alternatively insecticides specific for particular insect species/stages for example ovo-larvicides such as chofentezine, flubenzimine, hexythiazox and tetradifon, moltilicides such as dicofol or propargite, acaricides such as bromopropylate, chlorobenzilate, or growth regulators such as hydramethylon, cyromazin, methoprene, chlorofluazuron and diflubenzuron may also be included in the compositions.

Examples of suitable insecticide synergists for use in the compositions include piperonyl butoxide, sesamax, and dodecyl imidazole.

Suitable herbicides, fungicides and plant-growth regulators for inclusion in the compositions will depend upon the intended target and the effect required. An example of a rice selective herbicides which can be included is propanil, an example of a plant growth regulator for use in cotton is "Pix", and examples of fungicides for use in rice include blasticides such as blasticidin-S.

The ratio of the compound of the invention to the other active ingredient in the composition will depend upon a number of factors including type of target, effect required from the mixture etc.

However in general, the additional active ingredient of the composition will be applied at about the rate as it is usually employed, or at a slightly lower rate if synergism occurs.

The compounds of formula I and compositions comprising them have shown themselves active against a variety of insect and other invertebrate pests. They are particularly useful in controlling public health pests such as flies and cockroaches. Certain compounds of formula (I) and compositions comprising them are useful against pests in rice crops, such as rice hoppers. They may also be active against organophosphate and pyrethroid resistant strains of pests such as houseflies (Musca domestica). They may be effective in combating both susceptible and resistant strains of the pests in their adult, larval and intermediate stages of growth, and may be applied to the infested host animal by topical, oral or parenteral administration. The compounds also have nematocidal activity.

The following Examples illustrate various aspects of this invention. In the Preparations and Examples the products were usuallly identified and characterised by means of nuclear magnetic resonance spectroscopy and infra red spectroscopy. In each case where a product is specifically named its spectral characteristics are consistent with the assigned structure.

Preparation 1

This illustrates the preparation of 5-chloro-4-trifluoromethyl-2-pyridone.

A mixture of 4-trifluoromethyl-2-pyridone (10.2g, 62.5 mmol), N-chlorosuccinimide (8.4g, 62.9 mmol) and chloroform (45 ml) was heated at reflux for 2 hours. The reaction mixture was filtered and the filtrate evaporated in vacuo to give a white solid which was extracted with ethyl acetate. The ethyl acetate solution was washed with water and brine, dried over magnesium sulphate and evaporated in vacuo . The residue was recrystallised from a mixture of acetone and petrol to give the required compound (8.5g, mp. 173.5-174.5°C); δ ($d_6$ -DMSO) 7.94 (1H,s); 6.95 (1H,s).

Preparation 2

This illustrates the preparation of 5-bromo-4-trifluoromethyl-2-pyridone.

Bromine (6.3 ml, 0.122 mol) was added to a solution of 4-trifluoromethyl-2-pyridone (10g, 61 mmol) in acetic acid (20 ml). The reaction mixture was heated under reflux for two hours, allowed to cool, and poured into aqueous sodium thiosulphate solution. The aqueous mixture was extracted with ethyl acetate, washed with brine, saturated aqueous sodium bicarbonate and again with brine, dried over magnesium sulphate and evaporated in vacuo. The residue was recrystallised from a mixture of ethyl acetate and petrol to give the required compound (6.3g); ($CDCl_3$/$d_6$-DMSO) 7.72 (1H,s); 6.90 (1H,s).

Preparation 3

This illustrates the preparation of 3-bromo-2-chloro-5-trifluoromethylpyridine.

A mixture of 3-bromo-5-trifluoromethyl-2-pyridone (1 g, 4.13 mmol), phosphorus pentachloride (1.72 g, 8.2 mmol) and phosphorus oxychloride (2 ml) was heated under reflux for six hours. A further small portion of phosphorus pentachloride was added and heating continued for an additional six hours. The solution was poured into ice which was allowed to melt, and then extracted with ethyl acetate. The organic portion was washed with aqueous sodium bicarbonate solution, then brine, dried over magnesium sulphate and evaporated in vaco. The residue was purified by column chromotography on silica (1:1 diethyl ether - petrol as eluent) to give the desired compound as a clear liquid (660 mg); δ ($CDCl_3$) 8.62 (1H,s), 8.18 (1H,s).

Preparation 4

This illustrates the preparation of 2-chloro-3-nitro-5-trifluoromethylpyridine.

To an ice-cooled flask containing concentrated sulphuric acid was slowly added 5-trifluoromethyl-2-pyridone (20.14 g, 124 mmol). A cooled mixture of conc sulphuric acid (30 ml) and conc nitric acid (30 ml) was added slowly dropwise over 30 minutes, ensuring that the internal temperature was maintained below 25°C. The mixture was then warmed to 65°C, leading to a vigorous evolution of nitrogen dioxide. The reaction mixture was promptly cooled in ice and upon reaching room temperature was poured into ice (600 ml) with stirring.

The thawed mixture was extracted with ethyl acetate, dried with magnesium sulphate and evaporated in vacuo to give a sticky yellow solid which upon trituration with petrol gave 3-nitro-5-trifluoromethyl-2-pyridone (14.54 g). δ ($CDCl_3$ + $D_6$DMSO) 8.52 (1H,d), 8.01 (1H,d).

This product (14.40 g, 69.2 mmol) was slowly added to phosphorus oxychloride (75 ml) and the mixture warmed to 80°C until complete dissolution had occurred.

Phosphorus pentachloride (14.6 g) was added and the reaction heated at 100°C for two hours; excess phosphorus oxychloride was removed by evaporation in vacuo, and the residue poured onto ice. The thawed mixture was extracted with diethyl ether, dried over magnesium sulphate, and evaporated in vacuo to give a red oil which was purified by chromatography on silica with 30% diethyl ether-petrol eluent. The pure product (12.45 g) showed δ ($CDCl_3$) 8.92 (1H,s), 8.50 (1H,s).

EXAMPLE 1

This Example illustrates the preparation of 1-(3-chloro-5-trifluoromethyl-2-pyridyl)-4-trifluoromethyl-2-pyridone (Compound No. 1, Table I).

Sodium hydride (0.4g of a 50% dispersion in mineral oil, 8.3mmol) was washed with petroleum ether (40-60°C boiling range) in a dry flask flushed with nitrogen. Dimethylformamide (1ml) was added and the mixture gently stirred at room temperature whilst a solution of 4-trifluoromethyl-2-pyridone (1.21g, 7.4mmol) in dimethylformamide (10ml) was added dropwise.

After a further thirty minutes at room temperature 2-fluoro-3-chloro-5-trifluoromethylpyridine (1.51g, 7.6mmol) dissolved in dimethylformamide (5ml) was added and the mixture stirred at ambient temperature for sixteen hours.

The reaction mixture was evaporated under reduced pressure to give an oily residue which was poured into brine, acidified with dilute hydrochloric acid and extracted with ethyl acetate. The organic extracts were washed with brine, dried over magnesium sulphate and evaporated under reduced pressure to give a brown residue which was purified by dry column chromatography on silica with 6:1 petrol (40-60°C): diethylether eluent. The resulting white solid was recrystallised from a petrol (40-60°C)-diethyl ether mixture to give the required compound as neeldes (922mg, m.p. 140.8-141.7°C) $\delta$ ($d^6$-acetone) 8.90 (1H, m), 8.55 (1H, dm), 7.9 (1H,d), 6.85 (1H, m), 6.59 (1H, dd).

EXAMPLE 2

By the use of procedures similar to that illustrated in Example 1 the following compounds were prepared from the appropriate compounds of formula (II) wherein $R^{13}$ is fluoro and formula (III):

(i) 1-(3-chloro-5-trifluoromethyl-2-pyridyl)-5-chloro-4-trifluoromethyl-2-pyridone (Compound No. 2, Table I). The compound showed m.p. 159.1-160.3°C; $\delta$ ($d^6$-acetone) 8.95 (1H, m), 8.55 (1H, m), 8.1 (1H, s), 7.0 (1H, s).

(ii) 1-(3-chloro-5-trifluoromethyl-2-pyridyl)-5-bromo-4-trifluoromethyl-2-pyridone (Compound No. 3, Table I). The compound showed m.p. 174.4-175.2°C; $\delta$ ($d^6$ acetone) 8.90 (1H, m), 8.55 (1H, dm), 8.15 (1H, s), 7.0 (1H, s).

(iii) 1-(3-chloro-5-trifluoromethyl-2-pyridyl)-4,5-bis(trifluoromethyl)-2-pyridone (Compound No. 4, Table I). The compound showed m.p. 124.5-126.3°C; $\delta$ ($CDCl_3$) 8.82 (1H, m), 8.22 (1H, m), 7.94 (1H, m), 7.12 (1H, m).

(iv) 1-(3-chloro-5-trifluoromethyl-2-pyridyl)-4-difluorochloromethyl-2-pyridone (Compound No 6, Table I) except that the reaction mixture was heated at 60°C for 3.0 minutes. The compound showed mp 122-124.5°C; $\delta$ ($CDCl_3$) 8.76 (1H,dq) 8.16 (1H,dq), 7.43 (1H,dd), 6.90 (1H,m).

EXAMPLE 3

By the use of procedures similar to that illustrated in Example 1 the following compounds were prepared by reacting the appropriate compounds of formula (II) wherein $R^{13}$ is chloro with compounds of formula (III):

(i) 1-(3,6-dichloro-5-trifluoromethyl-2-pyridyl)-5-chloro-4-trifluoromethyl-2-pyridone (Compound No. 5, Table I). The compound showed $\delta$ ($CDCl_3$): 8.24 (1H, s), 7.68 (1H, s), 7.05 (1H, s).

(ii) 1-(3-bromo-5-trifluoromethyl-2-pyridyl)-4-trifluoromethyl-2-pyridone (Compound No 7, Table I) except that the reaction was heated at 90°C for sixteen hours. The compound showed $\delta$ ($CDCl_3$) 8.8 (1H,m), 8.35 (1H,m), 7.45 (1H,dm), 6.95 (1H,m), 6.45 (1H,dd).

(iii) 1-(3-nitro-5-trifluoromethyl-2-pyridyl)-4-trifluoromethyl-2-pyridone (Compound No 8, Table I). The compound showed mp 117-121.8°C; $\delta$ ($CDCl_3$) 9.07 (1H,s), 8.68 (1H,s), 7.93 (1H,d), 6.89 (1H,s), 6.59 (1H,d).

EXAMPLE 4

This illustrates the preparation of 1-(3-amino-5-trifluoromethyl-2-pyridyl)-4-trifluoromethyl-2-pyridone (Compound No 9, Table I). Compound 8 (2.64 g, 7.48 mmol) was dissolved in methanol (10 ml) and added to a solution of stannous chloride (4.26 g, 22.5 mmol) in conc hydrochloric acid (100 ml). The mixture was

stirred at ambient temperature (ca 25°C) for two hours, then poured into water and extracted with ethyl acetate. The organic extract was dried with magnesium sulphate and evaporated in vacuo to give the desired compound as an off-white solid: δ ($CDCl_3$) 8.03 (1H,s), 7.70 (1H,d), 7.58 (1H,s), 6.87 (1H,s), 6.51 (1H,dd), 5.60 (2H,br s).

Biological Data

The insecticidal activity of compounds of formula (I) is set out in the following Table II as a grading of A, B or C where A indicates that 80-100% mortality was observed, B indicates that 50-79% mortality was observed and C indicates that 0-49% mortality was observed. The tests were conducted by spraying a suitable support medium (eg, leaves of a suitable food plant, or filter paper) with a solution of the compound under test and placing the pests thereon. Assessment of mortality was made 72 hours after spraying. In the test the compounds were used in the form of aqueous composition prepared by dissolving the compound in mixture of solvents consisting of 1 part by volume of acetone and 1 part by volume of ethanol and diluting the solution with water containing 1% by volume of a wetting agent (Synperonic "NX - Synperonic" is a Registered Trade Mark) to give a final concentration of active ingredient of 500 ppm.

TABLE II

| COMPOUND | NL | MD | BG | HV | SE | DB |
|---|---|---|---|---|---|---|
| 1 | A | A | A | C | C | B |
| 2 | A | A | A | B | C | C |
| 3 | A | C | A | C | C | C |
| 4 | A | A | A | B | C | C |
| 5 | C | C | B | C | C | C |
| 6 | C | A | C | B | B | A |
| 7 | A | A | A | C | A | C |
| 8 | A | B | C | C | B | C |
| 9 | C | B | C | C | B | A |

TABLE III

| CODE LETTERS (Table III) | TEST SPECIES | SUPPORT MEDIUM/FOOD | TYPE OF TEST | DURATION (days) |
|---|---|---|---|---|
| NL | Nilaparvata lugens (brown plant hopper) | Rice plant | CT | 3 |
| SE | Spodoptera exigua | | RT | 3 |
| DB | Diabrotica balteata (rootworm larvae) | Filter paper/ maize seed | RT | 3 |
| BG | Blattella germanica (cockroach nymphs) | Plastic pot/ calf weenes pellets | RT | 3 |
| MD | Musca domestica (houseflies - adults) | Cotton wool/ sugar | CT | 1 |
| HV | Heliothis virescens | Cotton leaf | RT | 3 |

## Claims

1. A compound of formula (I)

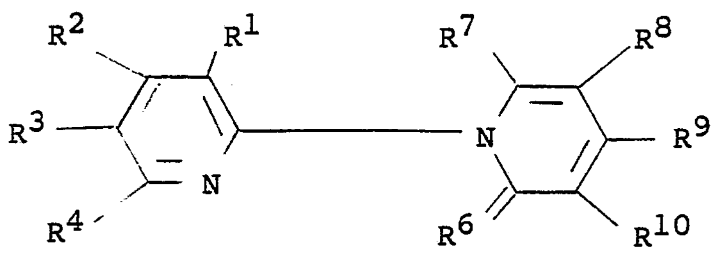

(I)

wherein $R^1$, $R^2$, $R^3$ and $R^4$ are independently selected from hydrogen, cyano, halogen, lower alkyl optionally substituted by halogen, lower alkoxy optionally substituted by halogen, lower alkenyl optionally substituted by halogen; amino and mono-or di-(lower alkyl) amino;
$R^6$ is oxygen or sulphur;
$R^7$, $R^8$ and $R^{10}$ are independently selected from hydrogen, halogen, cyano, lower alkyl optionally substituted by halogen, lower alkoxy optionally substituted by halogen, lower thioalkoxy optionally substituted by halo; and
$R^9$ is lower alkyl substituted by halogen or lower alkenyl optionally substituted by halogen.

2. A compound according to claim 1 wherein $R^9$ is lower alkyl substituted by fluorine or chlorine.

3. A compound according to claim 2 wherein $R^9$ is trifluoromethyl.

4. A compound of formula (IA)

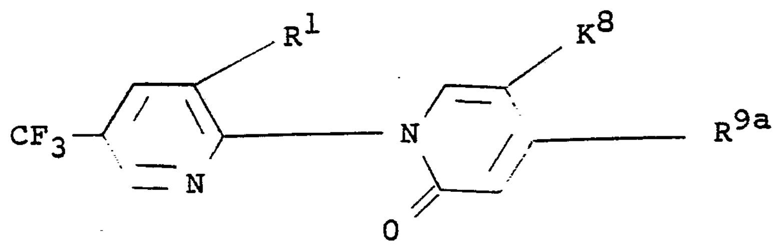

(IA)

wherein $R^1$ and $R^8$ are as defined in claim 1 and $R^{9a}$is lower alkyl substituted by halogen.

5. A compound according to claim 4 wherein $R^{9a}$ is trifluoromethyl.

6. A method for the preparation of a compound of formula (I) as defined in claim 1, which process comprises reacting a compound of formula (II)

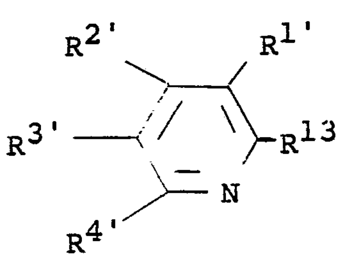

(II)

wherein $R^{1'}$, $R^{2'}$, $R^{3'}$ and $R^{4'}$ are $R^1$, $R^2$, $R^3$ and $R^4$ as hereinbefore defined or a precursor thereof and $R^{13}$ is a leaving group such as halo, with a compound of formula (III)

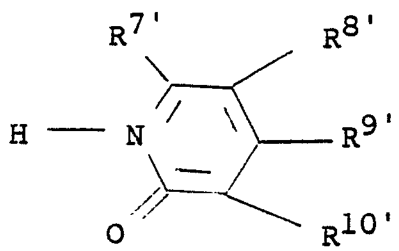

(III)

where $R^{7'}$, $R^{8'}$, $R^{9'}$ and $R^{10'}$ are $R^7$, $R^8$, $R^9$ or $R^{10}$ as hereinbefore defined or a precursor thereof; and thereafter is desired carrying out one or more of the following steps.

(i) converting a group $R^{1'}$, $R^{2'}$, $R^{3'}$, $R^{4'}$, $R^{5'}$, $R^{7'}$., $R^{8'}$, $R^{9'}$, $R^{10'}$ to $R^1$, $R^2$, $R^3$, $R^4$, $R^5$, $R^7$, $R^8$, $R^9$ or $R^{10}$ respectively; or

(ii) converting the carbonyl group to =S.

7. An insecticidal composition comprising a compound of formula (I) as defined in claim 1 in combination with a diluent or carrier .

8. A method of killing or controlling insect, acarine or nematode pests which comprising applying to the pests or to the locus thereof.

9. A compound of formula (VII)

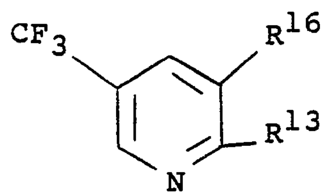

(VII)

wherein $R^{13}$ is a leaving group such as halogen and $R^{16}$ is bromo or nitro.

10. A compound of formula